(19) **Europäisches Patentamt / European Patent Office / Office européen des brevets**

(11) **EP 4 778 628 A1**

(12) **EUROPEAN PATENT APPLICATION**
published in accordance with Art. 153(4) EPC

(43) Date of publication:
**22.07.2026 Bulletin 2026/30**

(21) Application number: **24865185.3**

(22) Date of filing: **21.08.2024**

(51) International Patent Classification (IPC):
**B01J 23/888** (2006.01)    **C07B 61/00** (2006.01)
**C07C 51/235** (2006.01)    **C07C 57/055** (2006.01)

(52) Cooperative Patent Classification (CPC):
**B01J 23/888; C07B 61/00; C07C 51/235; C07C 57/04**

(86) International application number:
**PCT/JP2024/029661**

(87) International publication number:
**WO 2025/057681 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.09.2023  JP 2023150163**

(71) Applicant: **Nippon Kayaku Kabushiki Kaisha Tokyo 100-0005 (JP)**

(72) Inventors:
- **YASUDA, Shogo**
  **Sanyoonoda-shi, Yamaguchi 757-8686 (JP)**
- **MORISAWA, Kazeto**
  **Sanyoonoda-shi, Yamaguchi 757-8686 (JP)**
- **OKUMURA, Shigeki**
  **Sanyoonoda-shi, Yamaguchi 757-8686 (JP)**

(74) Representative: **Gille Hrabal Partnerschaftsgesellschaft mbB Patentanwälte Brucknerstraße 20 40593 Düsseldorf (DE)**

(54) **CATALYST FOR PRODUCTION OF UNSATURATED CARBOXYLIC ACID AND METHOD FOR PRODUCING UNSATURATED CARBOXYLIC ACID**

(57)    The present disclosure relates to a catalyst for producing an unsaturated carboxylic acid in which a catalytically active component has a composition represented by the following formula (1) and in which a width at half height of a peak at $22.2° \pm 0.3°$ in an X-ray diffraction pattern obtained using CuKα ray as an X-ray source is $0.65°$ or more and $1.60°$ or less. $(Mo)_{12}(V)_a(W)_b(Cu)_c(Sb)_d(X)_e(Y)_f(Z)_g(O)_h$ (1)

**FIG. 1**

EP 4 778 628 A1

**Description**

TECHNICAL FIELD

The present invention relates to a catalyst for producing an unsaturated carboxylic acid in a high yield by subjecting an unsaturated aldehyde to catalytic gas-phase oxidation in the presence of molecular oxygen or a molecular oxygen-containing gas, a production method therefor, and a method for producing an unsaturated carboxylic acid using the catalyst.

BACKGROUND ART

Acrylic acid is becoming increasingly important as a raw material for a water absorbent resin, an adhesive, and the like. Thus, in recent years, improved performance of a catalyst for producing acrylic acid by subjecting acrolein as a raw material to a catalytic gas-phase oxidation reaction has been demanded. Accordingly, various companies have made various improvements to catalysts that can be used to produce acrylic acid in a high yield and in a stable manner over a long period of time, and for example, the following proposals have been made.
PTLs 1 to 4 disclose improvements in catalyst composition or the like focusing on X-ray diffraction peaks of a catalytically active component. These catalysts have been proposed as a catalyst that achieves high activity and a high yield. Further, in PTLs 5 and 6, an improvement guideline for the purpose of improving the mechanical strength is shown, and the catalyst performance is improved through prevention of powdering during filling. In PTL 7, by adjusting the standard deviation of the particle diameter of the catalyst within a specific range, the long-term stability of the catalytic reaction is improved. PTL 8 proposes a method for producing a catalyst having both high catalyst performance and mechanical strength by controlling the relative centrifugal acceleration during formation using a tumbling granulator. PTL 9 proposes a method for producing a catalyst having a high yield by controlling the density of the formed catalyst body and the roughness of the catalyst surface. PTL 10 proposes a production method in which high activity and selectivity are obtained by controlling the crystal size in a specific range in a nanocrystalline molybdenum mixed oxide.
However, particularly in production of an unsaturated carboxylic acid, improvement of the catalytic activity is an important issue. This not only contributes to an increase in the yield but is also important from the viewpoint of catalyst life. For example, when the temperature of the reaction bath at the initial stage of the reaction decreases due to improvement in the catalytic activity in a plant using a catalyst, the energy cost for heating decreases. Moreover, because the thermal deterioration of the catalyst is reduced, the decrease in performance is small from a long-term viewpoint, and stable operation and a high yield can be achieved for a long time.
For the above reasons, a highly active catalyst and a method for producing the same are required.

CITATION LIST

PATENT LITERATURE

PTL 1: JPH08-299797A
PTL 2: JP2003-251184A
PTL 3: JP2015-120133A
PTL 4: JP2018-43197A
PTL 5: JP2001-79408A
PTL 6: WO2012/073584
PTL 7: JP2009-214105A
PTL 8: JP2015-96497A
PTL 9: WO2020/196150
PTL 10: JP2011-516378A

SUMMARY OF INVENTION

TECHNICAL PROBLEM

In view of the above circumstances, an object of the invention of the present application is to improve the catalytic activity of a catalyst for producing an unsaturated carboxylic acid by a catalytic gas-phase oxidation reaction from an unsaturated aldehyde as a raw material.

SOLUTION TO PROBLEM

As a result of intensive studies on the current situation and the object described above, the inventors of the present application have found that a specific parameter in an X-ray diffraction pattern contributes to improvement in activity and made the invention of the present application.

That is, the present invention relates to the following 1) to 6).

1)
A catalyst for producing an unsaturated carboxylic acid in which the catalytically active component has a composition represented by the following formula (1), and a width at half height of a peak at 22.2° ± 0.3° in an X-ray diffraction pattern obtained using CuKα ray as an X-ray source is 0.65° or more and 1.60° or less,

$$(Mo)_{12}(V)_a(W)_b(Cu)_c(Sb)_d(X)_e(Y)_f(Z)_g(O)_h \qquad (1)$$

(in the formula (1), Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of niobium, cerium, tin, chromium, manganese, iron, cobalt, samarium, germanium, titanium, and arsenic, a, b, c, d, e, f, g, and h represent atomic ratios of the respective elements, $0 < a \le 10.0$, $0 \le b \le 10.0$, $0 < c \le 6.0$, $0 \le d \le 10.0$, $0 \le e \le 0.50$, $0 \le f \le 1.0$, and $0 \le g < 6.0$ are satisfied with respect to molybdenum atom being 12, and h is the number of oxygen atoms necessary for satisfying the valence of each component.)

2) The catalyst for producing an unsaturated carboxylic acid according to 1) above in which in the formula (1), $1.0 \le a \le 5.0$, $0.50 \le b \le 3.0$, $0.50 \le c \le 3.0$, and $0 < d \le 2.0$ are satisfied.

3) The catalyst for producing an unsaturated carboxylic acid according to 1) or 2) above in which the catalytically active component is carried on an inert carrier.

4) The catalyst for producing an unsaturated carboxylic acid according to 3) above in which the inert carrier is silica, alumina, or a combination thereof.

5) A method for producing an unsaturated carboxylic acid using the catalyst for producing an unsaturated carboxylic acid according to any one of 1) to 4) above.

6) A method for producing an unsaturated carboxylic acid using a reaction tube filled with two or more types of the catalyst for producing an unsaturated carboxylic acid according to any one of 1) to 4) above in multiple layers.

ADVANTAGEOUS EFFECTS OF INVENTION

According to the present invention, the catalytic activity can be maintained high, when an unsaturated carboxylic acid, namely (meth)acrylic acid, is produced by subjecting an unsaturated aldehyde, preferably (meth)acrolein, as a raw material to a catalytic gas-phase oxidation reaction.

BRIEF DESCRIPTION OF DRAWINGS

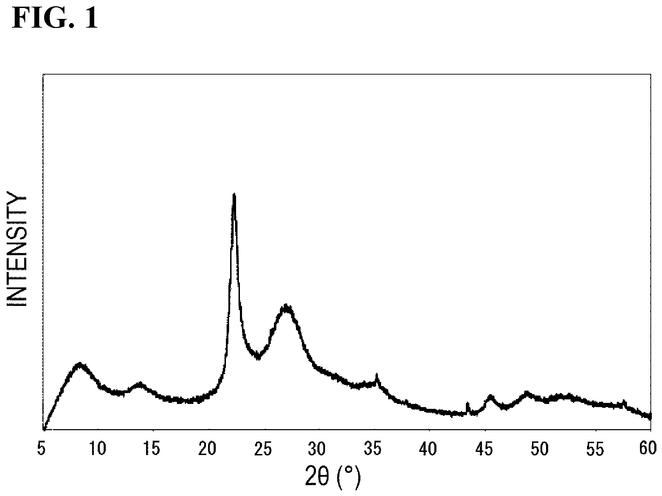

FIG. 1 is a figure showing the XRD chart of the catalytically active component of the catalyst 1 produced in Example 1.

DESCRIPTION OF EMBODIMENTS

[Width at Half Height of Peak at 22.2° ± 0.3°]

In the catalyst of the present invention, the width at half height of the peak at 22.2° ± 0.3° in an X-ray diffraction (XRD) pattern obtained using CuKα ray as an X-ray source is 0.65° or more and 1.60° or less.

In the present description, the width at half height means the full width at half maximum and is namely the width of the part where the intensity is 1/2 of the peak value in a quadratic function after fitting, which will be described later. Although details of how the width at half height of the peak at 22.2° ± 0.3° in the X-ray diffraction pattern affects the activity are unknown, it is considered that the specific crystal structure causes the oxidation reaction of (meth)acrolein because there is a clear relationship between the width at half height of the peak at 22.2° ± 0.3° and the catalytic activity.

Although most of the active components of the catalyst for producing (meth)acrylic acid of the present invention have an amorphous structure, it is considered that some of the crystals present exhibit high catalytic activity. The width at half height of the peak at 22.2° ± 0.3° in the X-ray diffraction pattern becomes small when the crystal having a diffraction plane at 22.2° ± 0.3° grows. It is considered that the oxidation reaction of (meth)acrolein proceeds inside the crystal structure, and it is considered that the activity of the catalyst improves because the number of reaction cites increases inside the crystal structure as the crystal grows. However, it is considered that the activity decreases when the crystal becomes too large, because physical resistance is generated and (meth)acrolein as a raw material does not easily pass through the inside of the crystal structure.

Further, the width at half height of the peak at 22.2° ± 0.3° is a value that increases or decreases depending on the number of crystals. As the proportion of the amorphous portion in the active components decreases and the number of crystals increases, the width at half height decreases because the diffraction plane increases, and the activity improves due to an increase in components with high activity.

In addition, the width at half height of the peak at 22.2° ± 0.3° is a value that increases or decreases depending on the strain of the crystal structure. In general, catalytic reaction is said to easily proceed in a part having a structural defect, and it is considered that a catalyst having a structural defect to some extent becomes a catalyst having a high activity also in a catalyst for producing (meth)acrylic acid. When there is a structural defect, strain generates in the crystal structure, and the width at half height of the peak at 22.2° ± 0.3° in the X-ray diffraction pattern becomes a large value.

Overall, the inventors of the present invention believe that a catalyst in which the degree of crystal growth and the structural strain are in certain ranges and in which the number of crystals is high is a preferable form as a catalyst for producing (meth)acrylic acid, and the inventors have found that such a catalyst can be obtained by adjusting the width at half height of the peak at 22.2° ± 0.3° in the X-ray diffraction pattern at 0.65° or more and 1.60° or less.

The width at half height in the present invention is 0.65° or more and 1.60° or less, but further preferable values of the lower limit are 0.70°, 0.75°, 0.80°, 0.85°, 0.88°, 0.90°, 0.92°, 0.94°, and 0.96° in this order, and 0.98° is particularly preferable. Similarly, preferable values of the upper limit are 1.55°, 1.50°, 1.47°, 1.45°, 1.40°, 1.35°, and 1.33° in this order, and 1.31° is particularly preferable. Therefore, the width at half height of the peak at 22.2° ± 0.3° is preferably 0.70° or more and 1.60° or less, more preferably 0.75° or more and 1.60° or less, more preferably 0.80° or more and 1.60° or less, more preferably 0.85° or more and 1.55° or less, more preferably 0.88° or more and 1.50° or less, more preferably 0.90° or more and 1.47° or less, more preferably 0.92° or more and 1.45° or less, more preferably 0.94° or more and 1.40° or less, more preferably 0.96° or more and 1.35° or less, and most preferably 0.98° or more and 1.31° or less. The width at half height of the peak at 22.2° ± 0.3° may be 0.65° or more and 1.47° or less, may be 0.65° or more and 1.45° or less, may be 0.65° or more and 1.40° or less, may be 0.85° or more and 1.60° or less, may be 0.88° or more and 1.60° or less or may be 0.90° or more and 1.60° or less.

Here, the method for measuring the X-ray diffraction angle (2θ) may be, for example, measurement of the X-ray diffraction angle (2θ) using Ultima IV manufactured by Rigaku Holdings Corporation under the condition of using X-ray CuKα ray (λ = 0.154 nm), output of 40 kV and 30 mA, a measurement range of 5 to 60°, and a measurement speed of 3° per minute, but the method is not limited thereto unless deviating from the measurement principle.

[Calculation Method of Width at Half Height of Peak at 22.2° ± 0.3°]

In calculating the width at half height, it is preferable to use data that are not subjected to processing such as smoothing of the baseline and to use OriginPro manufactured by LightStone Corporation as the analysis software. As an analysis method, a straight line passing through the minimum value of 2θ = 16.0° to 18.0° and the minimum value of 2θ = 39.0° to 42.0° in an X-ray diffraction pattern is used as a baseline, and quadratic functions are fitted to the peaks above the baseline. The full width at half maximum of a quadratic function after fitting having a vertex in the range of 2θ = 22.2° ± 0.3° is read as the width at half height of the peak of 2θ = 22.2° ± 0.3° in the X-ray diffraction pattern.

[Width at Half Height of Peak at 8.3° ± 0.5°]

In the catalyst of the present invention, further preferably, the width at half height of the peak at 8.3° ± 0.5° is greater than 0 and 2.82° or less from the viewpoint of catalytic activity. The upper limit of the width at half height of the peak at 8.3° ± 0.5° is further preferably 2.75°, 2.70°, 2.65°, 2.60°, 2.55°, 2.50°, 2.45°, or 2.40° in this order and is particularly preferably 2.37°. Similarly, the lower limit is further preferably 1.00°, 1.50°, 2.00°, 2.10°, or 2.20° in this order and is particularly preferably 2.30°. Therefore, the width at half height of the peak at 8.3° ± 0.5° is more preferably greater than 0 and 2.75° or less, more preferably greater than 0 and 2.70° or less, more preferably greater than 0 and 2.65° or less, more preferably 1.00° or more and 2.60° or less, more preferably 1.50° or more and 2.55° or less, more preferably 2.00° or more and 2.50° or less, more preferably 2.10° or more and 2.45° or less, more preferably 2.20° or more and 2.40° or less, and most preferably 2.30° or more and 2.37° or less.

[Calculation Method of Width at Half Height of Peak at 8.3° ± 0.5°]

The calculation method is the same as the calculation method of the width at half height of the peak at 22.2° ± 0.3° except that the width at half height of a quadratic function having a vertex in the range of $2\theta = 8.3° \pm 0.5°$ is read as the width at half height of the peak, of the quadratic functions after fitting.

[Width at Half Height of Peak at 26.7° ± 0.3°]

In the catalyst of the present invention, further preferably, the width at half height of the peak at 26.7° ± 0.3° is 4.66° or more and 7.40° or less from the viewpoint of catalytic activity. The upper limit of the width at half height of the peak at 26.7° ± 0.3° is further preferably 7.30°, 7.25°, 7.20°, 7.00°, 6.90°, 6.80°, 6.70°, 6.60°, 6.50°, 6.40°, 6.30°, 6.20°, 6.15°, or 6.10° in this order and is particularly preferably 6.05°. Similarly, the lower limit is further preferably 4.80°, 5.00°, 5.05°, 5.10°, 5.20°, 5.30°, 5.40°, 5.45°, 5.50°, 5.60°, 5.70°, 5.80°, or 5.90° in this order and is particularly preferably 5.95°. Therefore, the width at half height of the peak at 26.7° ± 0.3° is more preferably 4.66° or more and 7.30° or less, more preferably 4.66° or more and 7.25° or less, more preferably 4.80° or more and 7.20° or less, more preferably 5.00° or more and 7.00° or less, more preferably 5.10° or more and 6.90° or less, more preferably 5.20° or more and 6.80° or less, more preferably 5.30° or more and 6.70° or less, more preferably 5.40° or more and 6.60° or less, more preferably 5.45° or more and 6.50° or less, more preferably 5.50° or more and 6.40° or less, more preferably 5.60° or more and 6.30° or less, more preferably 5.70° or more and 6.20° or less, more preferably 5.80° or more and 6.15° or less, more preferably 5.90° or more and 6.10° or less, and most preferably 5.95° or more and 6.05° or less.

[Calculation Method of Width at Half Height of Peak at 26.7° ± 0.3°]

The calculation method is the same as the calculation method of the width at half height of the peak at 22.2° ± 0.3° except that the width at half height of a quadratic function having a vertex in the range of $2\theta = 26.7° \pm 0.3°$ is read as the width at half height of the peak, of the quadratic functions after fitting.

[Width at Half Height of Peak at 45.4° ± 0.3°]

In the catalyst of the present invention, further preferably, the width at half height of the peak at 45.4° ± 0.3° is 0.64° or more and 1.03° or less from the viewpoint of catalytic activity. The upper limit of the width at half height of the peak at 45.4° ± 0.3° is further preferably 1.00°, 0.99, or 0.98 in this order and is particularly preferably 0.97°. Similarly, the lower limit is further preferably 0.65°, 0.67°, 0.70°, 0.75°, 0.80°, 0.85°, or 0.90° in this order and is particularly preferably 0.95°. Therefore, the width at half height of the peak at 45.4° ± 0.3° is more preferably 0.65° or more and 1.03° or less, more preferably 0.67° or more and 1.03° or less, more preferably 0.70° or more and 1.03° or less, more preferably 0.75° or more and 1.03° or less, more preferably 0.80° or more and 1.00 or less, more preferably 0.85° or more and 0.99° or less, more preferably 0.90° or more and 0.98° or less, and most preferably 0.95° or more and 0.97° or less.

[Calculation Method of Width at Half Height of Peak at 45.4° ± 0.3°]

The calculation method is the same as the calculation method of the width at half height of the peak at 22.2° ± 0.3° except that the width at half height of a quadratic function having a vertex in the range of $2\theta = 45.4° \pm 0.3°$ is read as the width at half height of the peak, of the quadratic functions after fitting.

[Catalyst Composition]

The catalyst according to the present invention has a composition represented by the following formula (1).
[Formula (1)]

$$(Mo)_{12}(V)_a(W)_b(Cu)_c(Sb)_d(X)_e(Y)_f(Z)_g(O)_h \qquad (1)$$

(In the formula, Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of bismuth, tellurium, silver, selenium, silicon, aluminum, boron, niobium, cerium, tin, chromium, manganese, iron, cobalt, nickel, samarium, germanium, zirconium, titanium, chromium, tantalum, lead, indium, sulfur, palladium, gallium, lanthanum, and arsenic. a, b, c, d, e, f, g, and h represent the atomic ratios of the respective elements and satisfy $0 < a \leq 10.0$, $0 \leq b \leq 10.0$, $0 < c \leq 6.0$, $0 \leq d \leq 10.0$, $0 \leq e \leq 0.50$, $0 \leq f \leq 1.0$, and $0 \leq g < 6.0$, with respect to molybdenum atom being 12. h is the number of oxygen atoms necessary for satisfying the valence of each component.)

In the above formula (1), preferred ranges of a to g are as follows.

The lower limit of a is 0.20, 0.50, 0.80, 1.0, 1.5, 2.0, 2.2, or 2.4 in order of desirability and is most desirably 2.6, and the upper limit of a is 9.0, 8.0, 7.0, 6.0, 5.0, 4.5, 4.0, 3.5, 3.3, or 3.1 in order of desirability and is most desirably 2.9. That is, the range of a is preferably $0.20 \le a \le 9.0$, more preferably $0.20 \le a \le 8.0$, more preferably $0.20 \le a \le 7.0$, more preferably $0.50 \le a \le 6.0$, more preferably $0.80 \le a \le 5.0$, more preferably $1.0 \le a \le 4.5$, more preferably $1.5 \le a \le 4.0$, more preferably $2.0 \le a \le 3.5$, more preferably $2.2 \le a \le 3.3$, more preferably $2.4 \le a \le 3.1$, and most preferably $2.6 \le a \le 2.9$.

The lower limit of b is 0.10, 0.20, 0.30, 0.40, 0.50, or 0.60 in order of desirability and is most desirably 0.70, and the upper limit of b is 9.0, 8.0, 7.0, 6.0, 5.0, 4.0, 3.0, 2.5, 2.0, 1.5, or 1.3 in order of desirability and is most desirably 1.1. That is, the range of b is preferably $0.10 \le b \le 9.0$, more preferably $0.10 \le b \le 8.0$, more preferably $0.10 \le b \le 7.0$, more preferably $0.10 \le b \le 6.0$, more preferably $0.10 \le b \le 5.0$, more preferably $0.10 \le b \le 4.0$, more preferably $0.20 \le b \le 3.0$, more preferably $0.30 \le b \le 2.5$, more preferably $0.40 \le b \le 2.0$, more preferably $0.50 \le b \le 1.5$, more preferably $0.60 \le b \le 1.3$, and most preferably $0.70 \le b \le 1.1$.

The lower limit of c is 0.10, 0.20, 0.30, 0.40, 0.50, 0.60, 0.70, 0.80, 0.90, or 1.0 in order of desirability and is most desirably 1.3, and the upper limit of c is 5.0, 4.0, 3.0, 2.5, 2.0, or 1.8 in order of desirability and is most desirably 1.6. That is, the range of c is preferably $0.10 \le c \le 5.0$, more preferably $0.20 \le c \le 5.0$, more preferably $0.30 \le c \le 5.0$, more preferably $0.40 \le c \le 5.0$, more preferably $0.50 \le c \le 5.0$, more preferably $0.60 \le c \le 4.0$, more preferably $0.70 \le c \le 3.0$, more preferably $0.80 \le c \le 2.5$, more preferably $0.90 \le c \le 2.0$, more preferably $1.0 \le c \le 1.8$, and most preferably $1.3 \le c \le 1.6$.

The lower limit of d is 0.11, 0.15, 0.18, 0.20, 0.25, 0.30, or 0.35 in order of desirability and is most desirably 0.40, and the upper limit of d is 9.0, 8.0, 7.0, 6.0, 5.0, 4.0, 3.0, 2.5, 2.0, 1.5, or 1.0 in order of desirability and is most desirably 0.70. That is, the range of d is preferably $0.11 \le d \le 9.0$, more preferably $0.11 \le d \le 8.0$, more preferably $0.11 \le d \le 7.0$, more preferably $0.11 \le d \le 6.0$, more preferably $0.11 \le d \le 5.0$, more preferably $0.15 \le d \le 4.0$, more preferably $0.18 \le d \le 3.0$, more preferably $0.20 \le d \le 2.5$, more preferably $0.25 \le d \le 2.0$, more preferably $0.30 \le d \le 1.5$, more preferably $0.35 \le d \le 1.0$, and most preferably $0.40 \le d \le 0.70$.

The upper limit of e is 0.40, 0.30, 0.20, or 0.10 in order of desirability. That is, the range of e is $0 \le e \le 0.40$, $0 \le e \le 0.30$, or $0 \le e \le 0.20$ in order of preference, and the most preferable range is $0 \le e \le 0.10$.

The upper limit of f is 0.80, 0.50, 0.20, or 0.15 in order of desirability and is most desirably 0.10. That is, the range of f is $0 \le f \le 0.80$, $0 \le f \le 0.50$, $0 \le f \le 0.20$, or $0 \le f \le 0.15$ in order of preference, and the most preferable range is $0 \le f \le 0.10$.

The upper limit of g is 5.0, 4.0, 3.0, 2.0, or 1.0 in order of desirability. That is, the range of g is $0 \le g \le 5.0$, $0 \le g \le 4.0$, $\le g \le 3.0$, or $\le g \le 2.0$ in order of preference, and the most preferable range is $\le g \le 1.0$.

Here, a case in which e, f, and g are 0 is a particularly preferable aspect.

When the catalyst of the present invention is used in a reaction that produces a corresponding unsaturated carboxylic acid from an unsaturated aldehyde such as acrolein and methacrolein as a raw material, particularly in a reaction that produces acrylic acid by subjecting acrolein to catalytic gas-phase oxidation with molecular oxygen or a molecular oxygen-containing gas, improvement in the catalytic activity and reduction in the differential pressure can be achieved, and the use is very effective, as compared with a known method. Also in a process of a partial oxidation reaction accompanied by heat generation, the effect of improving the stability due to reduction of the hot spot temperature or the like can be expected. Further, the catalyst of the present invention is also effective in reducing byproducts that adversely influence the environment and the quality of the final product, such as carbon monoxide (CO), carbon dioxide ($CO_2$), acetaldehyde, acetic acid, and formaldehyde.

[Method for Producing Catalyst, etc.]

Specific steps for obtaining the catalyst according to the present invention are exemplified below.

Step a) preparation

Examples of the raw materials of the elements constituting the catalyst include those shown below.

When ammonium molybdate is used as a molybdenum component raw material, a high-performance catalyst can be obtained.

As raw materials for tungsten, vanadium, antimony, copper and other elements, typically, an oxide, or an ammonium salt, a carbonate, a sulfate, an organic acid salt, a hydroxide, a metal powder, or the like which can be converted into an oxide by high heat, or a mixture thereof can be used. Preferable raw materials vary depending on the types of elements, and further, the width at half height of the peak at $22.2° \pm 0.3°$ in the XRD pattern can also be adjusted thereby. For example, an ammonium salt is preferable in the case of tungsten or vanadium, and a sulfate is preferable in the case of copper. In the case of an antimony raw material, a compound in which the valence of antimony is three is preferable, and salts of acetic acid, carbonic acid, tartaric acid, oxalic acid, and other carboxylic acids are particularly preferable. After a vanadium raw material, a molybdenum raw material, a tungsten raw material, and an antimony raw material are mixed in a desired proportion in an aqueous solvent at 20 to 95°C and stirred with heating for about an hour, an X component raw material, a Y component raw material, and a Z component raw material according to the need are added, and an aqueous solution obtained by dissolving a copper raw material is added at the end. An aqueous solution or slurry containing the catalyst components is obtained, which is hereinafter referred to as a prepared liquid (A). When a metal powder is used as a raw material, the metal powder is preferably added at the same timing as the antimony raw material, and when a copper raw material in which the valence of copper is two is used as the copper raw material, the copper raw material is preferably added after the antimony raw material and the metal powder are added. When the copper raw material in which the valence is two is added before the antimony raw material or the metal powder, a crystal having low activity is generated, which leads to a decrease in the catalyst performance.

Here, the prepared liquid (A) is not always necessary to contain all the catalyst constituent elements, and some elements or some amounts thereof may be added in the subsequent steps. In addition, in the case of adding an amount of water for dissolving the component raw materials or an acid such as sulfuric acid, nitric acid, hydrochloric acid, tartaric acid, and acetic acid for dissolution when preparing the prepared liquid (A), unless the sufficient acid concentration in the aqueous solution to dissolve the raw materials is suitable, for example, in the range of 5 mass% to 99 mass%, the prepared liquid (A) sometimes becomes in the form of a clay-like lump, and this does not result in an excellent catalyst. Therefore, the form of the obtained prepared liquid (A) is preferably an aqueous solution or slurry as an excellent catalyst can be obtained.

Step b) drying

Next, the prepared liquid (A) obtained above is dried to form a dry powder. The drying method is not limited as long as it is a method capable of completely drying the prepared liquid (A), and examples thereof include drum drying, freeze drying, spray drying, and evaporation to dryness. Among these, in the present invention, spray drying is particularly preferred since the slurry can be dried into a powder or granules in a short period of time. The drying temperature in the spray drying varies depending on a concentration of the slurry, a liquid feeding rate, or the like, and the temperature at the outlet of a dryer is typically 70°C to 150°C. In addition, drying is preferably performed such that the average particle diameter of the dry powder obtained at this time is 20 to 700 $\mu$m. In this way, a dry powder (B) is obtained.

Step c) preliminary calcination

When the obtained dry powder (B) is calcined under air circulation at 200°C to 500°C, preferably at 300°C to 400°C, the formability, the mechanical strength, and the catalyst performance of the catalyst tend to improve. The calcination time is preferably 1 hour to 12 hours. In this way, a preliminarily calcined product (C) is obtained.

Step d) pulverization

The obtained preliminarily calcined product (C) is obtained as a solid matter (D) in which the dry powder (B) is aggregated by preliminary calcination. The solid matter (D) is pulverized to obtain a preliminarily calcined powder (E) required in the next forming step. The pulverization method is not particularly restricted, but examples thereof include a roller mill, a jet mill, a hammer mill, a ball mill, and a vibration mill. The average particle diameter (pre-calcined median diameter) of the preliminarily calcined powder (E) obtained at this time is preferably 50 $\mu$m or less, more preferably 40 $\mu$m or less, further preferably 30 $\mu$m or less, and particularly preferably 25 $\mu$m or less.

In the present description of this application, the preliminarily calcined powder (E) after pulverization is described as a catalyst precursor, but when there is no aggregation in the stage of the preliminarily calcined product (C) and the preliminarily calcined product (C) can be used without going through the pulverization step, the preliminarily calcined product (C) may be used as a catalyst precursor.

Step e) forming

The forming method is not particularly limited, but in the case of forming into a cylindrical shape or a ring shape, a method using a tablet forming machine, an extrusion forming machine, or the like is preferred. A case of forming into a spherical shape is further preferable, and the preliminarily calcined powder (E) may be formed into a spherical shape using a forming machine, but a method of carrying the preliminarily calcined powder (E) (containing a forming aid and a strength improver if necessary) on a carrier such as inert ceramic is preferable. Here, as a carrying method, a tumbling granulation method, a method using a centrifugal fluid coating device, a wash coating method, and the like are widely known, and the method is not particularly limited as long as the preliminarily calcined powder (E) can be uniformly carried on the carrier. In consideration of the production efficiency of the catalyst and the performance of the prepared catalyst, more preferable is a method of rotating a flat or uneven disc at a high speed in a device that has the disc at the bottom of a fixed columnar container, to vigorously agitate a carrier charged in the container by rotation and revolution movements of the carrier itself, and carrying the powder component on the carrier by adding, to the container, the preliminarily calcined powder (E), and if necessary a forming aid and/or a strength improver, and a pore-forming agent. A tumbling granulation method is most preferable. A binder is preferably used for carrying. Specific examples of the binder to be used include water, ethanol, methanol, propanol, a polyhydric alcohol, polyvinyl alcohol that is a polymer-based binder, and a silica sol aqueous solution that is an inorganic binder. Ethanol, methanol, propanol, and a polyhydric alcohol are preferred, a diol such as ethylene glycol and a triol such as glycerin are more preferred. When an appropriate amount of an aqueous glycerin solution is used, the formability becomes excellent, and a high-performance catalyst having high mechanical strength can be obtained. Specifically, a particularly high-performance catalyst can be obtained when an aqueous solution of glycerin having a concentration of 5 mass% or more is used. The amount of these binders to be used is typically 2 to 80 parts by mass with respect to 100 parts by mass of the preliminarily calcined powder (E). An inert carrier of about 2 to 8 mm is typically used, and the preliminarily calcined powder (E) is carried thereon. The carrying ratio is determined in consideration of the catalyst usage conditions, for example, reaction conditions such as the space velocity of the reaction raw materials and the concentrations of the raw materials and is typically 20 mass% to 80 mass%. Here, the carrying ratio is expressed as in the following equation (4) when the forming aid, the strength improver, and the like used in forming are included. Thus, a formed body (F) is obtained.

In addition, as found by the present inventors, the catalyst of the invention of this case sometimes has poor mechanical strength. Therefore, it is preferable to add inert inorganic fibers as a strength improver during carrying and forming. The amount of the fibers to be used is typically 1 to 30 parts by mass with respect to 100 parts by mass of the preliminarily calcined powder (E).

[Carrying]

The catalyst in which the preliminarily calcined product (C), which is obtained by preliminary calcination after preparation of the catalytically active component, or the preliminarily calcined powder (E) after pulverization, which is obtained by further subjecting the preliminarily calcined product (C) to a pulverization step, is carried on an inert carrier has a particularly excellent effect.

As the material of the inert carrier, known materials such as alumina, silica, titania, zirconia, niobia, silica-alumina, silicon carbide, carbide, and a mixture thereof can be used. The particle diameter, the water absorption, the mechanical strength, the crystallinity and the mixing ratio of each crystal phase, and the like of the inert carrier are not particularly limited, and appropriate ranges should be selected in consideration of the final catalyst performance, the formability, the production efficiency, and the like. The shape of the inert carrier is preferably spherical but is not particularly limited, and a pellet, a ring, or the like is used. The mixing proportion of the carrier and the preliminarily calcined powder is typically calculated as a carrying ratio based on the charged mass of each raw material according to the following equation (4). When it is clear that the additives such as the forming aid and the strength improver used remain in the catalyst even after the main calcination, the additives are included in the total amount (denominator).

[Equation (4)]

$$\text{Carrying ratio (mass\%)} = \frac{\text{(mass of preliminarily calcined powder used for forming)}}{\text{(mass of preliminarily calcined powder used for forming)} + \text{(mass of carrier used for forming)}} \times 100 \quad (4)$$

The upper limit of the carrying ratio is preferably 80 mass% and is 70, 60, 55, 50, 45, or 40 mass% in order of preference.

The lower limit thereof is preferably 10 mass% and is 15, 18, or 20 mass% in order of preference. That is, the carrying ratio is preferably 10 mass% or more and 80 mass% or less, more preferably 10 mass% or more and 70 mass% or less, more preferably 10 mass% or more and 60 mass% or less, more preferably 10 mass% or more and 55 mass% or less, more preferably 15 mass% or more and 50 mass% or less, and more preferably 18 mass% or more and 45 mass% or less, and the most preferable range is 20 mass% or more and 40 mass% or less.

[Inorganic Fiber]

The catalyst of the present invention preferably contains inorganic fibers for the purpose of improving the mechanical strength or the like. The material of the inorganic fibers is not particularly limited, but for example, glass fibers, ceramic fibers, metal fibers, mineral fibers, carbon fibers, various whiskers, and the like can be used. Of these, glass fibers treated with a silane-based chemical product are particularly preferable.

The fiber length thereof is not particularly limited as long as it does not impair the effects of the present invention, but the average fiber length is preferably about 1 to 1000 $\mu$m, and more preferably about 10 to 500 $\mu$m.

Further, two or more types of the inorganic fibers can be used in combination, and two or more types having different materials or two types of a same material having different average fiber lengths may be used.

Step f) main calcination

When the formed body (F) is calcined at a temperature of 100 to 450°C for about 1 to 12 hours, the catalytic activity and the useful yield tend to improve. The calcination temperature is preferably 270°C or higher and 420°C or lower, and more preferably 350°C or higher and 400°C or lower. Air is convenient and preferred as the gas to be circulated. In addition, nitrogen, carbon dioxide, argon, helium, a nitrogen oxide-containing gas for creating a reducing atmosphere, an ammonia-containing gas, a hydrogen gas, and a mixture thereof can be also used as the inert gas. In this way, a catalyst (G) is obtained.

[Adjustment Method of Width at Half Height of Peak at 22.2° ± 0.3°]

The value of the width at half height above can be adjusted by changing the types and the proportions of the raw materials used in the step a), the spray drying condition in the step b), the calcination temperature, the time, and the atmosphere in the step c), the pulverization method and the median diameter after pulverization in the step d), and the relative centrifugal acceleration, the carrying ratio, the type of the binder, the addition position of the binder, and the like in the step e). However, it is difficult to greatly change the value by changing a single condition, and the value can be achieved by optimizing two or more conditions. Some examples will be described below.

<Types and Proportions of Raw Materials Used in Step a)>

The width at half height of the peak at 22.2° ± 0.3° in the XRD pattern can be adjusted by changing the types of the raw materials used. In particular, it is preferable to change a compound which can function as a reducing agent. The width at half height becomes large when a trivalent antimony compound is used, and the width at half height becomes small when a metal powder of molybdenum or copper in which the oxidation number is zerovalent or an organic material such as oxalic acid and citric acid is used. Further, the width at half height can also be controlled by adjusting the composition ratio of the metal elements. In the catalyst of the present invention, molybdenum and vanadium are used as the basic skeleton, and as the amounts of other elements increase, strain generates in the crystal structure, resulting in a tendency towards a large width at half height. In particular, because tungsten and antimony atoms cause strong strain in the crystal structure, the width at half height tends to be large as higher amounts of these raw materials are used, while the width at half height tends to be small when a high amount of the copper raw material is used. From the viewpoint, for example, the atomic ratio d of antimony with respect to molybdenum atom being 12 in the formula (1) may be $0 < d < 1.0$, may be $0.25 \leq d \leq 0.75$ or may be $0.25 \leq d \leq 0.50$.

<Calcination Temperature, Time, and Atmosphere in Step c)>

The calcination temperature is preferably 200°C to 500°C as described above. The width at half height tends to be small when the calcination temperature is high because the crystal growth is promoted, while the width at half height becomes large when the temperature is too high or the time is too long because the number of crystals is reduced by thermal decomposition. Therefore, from the viewpoint of adjusting the width at half height, the calcination temperature is preferably lower than 400°C, and the time is preferably less than six hours.
The gas atmosphere during calcination may be the atmosphere, but because the crystal growth is inhibited when there are oxygen molecules, the width at half height can be made small through calcination in an inert gas atmosphere such as nitrogen or argon or a reducing gas atmosphere such as hydrogen and ammonia.

<Pulverization Method, Median Diameter, and Centrifugal Acceleration in Step d)>

The width at half height can be controlled also by the method of the step d). It is easy to adjust the width at half height when a ball mill is used, and the width at half height can be adjusted also by adjusting the median diameter of the preliminarily calcined powder to a certain value or less. The median diameter of the preliminarily calcined powder is preferably 50 $\mu$m or less. Further, the width at half height can be adjusted by changing the relative centrifugal acceleration in the case where the step e) is tumbling granulation depending on the median diameter of the preliminarily calcined powder, and the relative centrifugal acceleration may be set to 10 G or more when the median diameter is smaller than 30 $\mu$m.

[Use of Catalyst]

In the method for producing an unsaturated carboxylic acid such as acrylic acid using the catalyst of the present invention, the method for circulating the raw material gas may be a normal single flow method or a recycling method, and the method can be carried out under generally used conditions and is not particularly limited. For example, a mixed gas containing 1 to 10 vol% and preferably 4 to 9 vol% of a starting raw material substance as an ideal gas, 3 to 20 vol% and preferably 4 to 18 vol% of molecular oxygen, 0 to 60 vol% and preferably 4 to 50 vol% of water vapor, and 20 to 80 vol% and preferably 30 to 75 vol% of an inert gas such as carbon dioxide and nitrogen is introduced, at 200 to 450°C under normal pressure to pressure of 10 atm at a space velocity of 300 to 5000 h$^{-1}$, into the catalyst of the present invention packed in a reaction tube, and the reaction is carried out.

In the method for producing an unsaturated carboxylic acid, one type of catalyst may be used alone, or different types of catalysts may be used for multilayer filling depending on the conditions to be used. That is, in an adoptable method, a plural number n of catalyst layers divided and formed in the direction of the raw material gas flow of the reaction tube are provided, and the plurality of types of catalysts are arranged in such a manner that the activity increases from the raw material inlet portion toward the outlet portion in the direction of the raw material gas flow. The number n of the divided layers is not particularly limited but is typically two to five, and preferably two or three. The different types of catalysts not only mean a case where the compositions of the catalysts are different alone but also include a case where the carrying ratios to the inert carrier are different or a case where the dilution rates are different. In addition, a method in that the catalyst of the present invention, which is highly activated by adjusting the width at half height, is disposed on the raw material gas outlet side and in that a catalyst that is relatively low in activity is disposed on the raw material gas inlet side can also be adopted.

Example

Hereinafter, the present invention will be described in more detail with reference to Examples. In the Examples, the raw material conversion rates, the yields, and the selectivity were calculated according to the following equations.

Raw material conversion rate (%) = (number of moles of reacted acrolein/number of moles of supplied acrolein) $\times$ 100

Yield (%) = (number of moles of produced acrylic acid)/(number of moles of supplied acrolein) $\times$ 100

Selectivity (%) = (number of moles of produced acrylic acid)/(number of moles of reacted acrolein) $\times$ 100

Although Examples are shown below by giving specific examples, the present invention is not limited to Examples unless it deviates from the spirit thereof. Here, the widths at half height of $2\theta = 22.2°$ shown in the Examples are the values obtained by measuring the X-ray diffraction angles ($2\theta$) using Ultima IV manufactured by Rigaku Holdings Corporation under the condition of using X-ray CuK$\alpha$ ray ($\lambda = 0.154$ nm), output of 40 kV and 30 mA, a measurement range of 5 to 60°, and a measurement speed of 3° per minute and analyzing the obtained data using OriginPro 2022b manufactured by LightStone Corporation. As an analysis method, as described in the preceding paragraph, a straight line passing through the minimum value of $2\theta = 16.0°$ to $18.0°$ and the minimum value of $2\theta = 39.0°$ to $42.0°$ in an X-ray diffraction pattern was used as a baseline, and quadratic functions were fitted to the peaks above the baseline. The width at half height of a quadratic function after fitting having a vertex in the range of $2\theta = 22.2° \pm 0.3°$ was read as the width at half height of the peak of $2\theta = 22.2° \pm 0.3°$ in the X-ray diffraction pattern.

[Example 1]

<Production of Catalyst 1>

Ammonium molybdate, ammonium paratungstate, ammonium metavanadate, and antimony acetate were weighed in such a manner that the composition with respect to $Mo_{12}$ became $V_{2.7}W_{0.8}Cu_{1.5}Sb_{0.50}$, and the materials were mixed in an aqueous solvent of 5.2 times the mass of the ammonium molybdate heated to 95°C. Then, copper sulfate was added to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). To the preliminarily calcined powder (E), 5 mass% of crystalline cellulose and 5 mass% of glass fibers having an average fiber length of 150 $\mu$m were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for formation, and the centrifugal acceleration was 26.0 G. Next, main calcination was performed under the conditions of 390°C and four hours to obtain a spherical catalyst 1 of the present invention.

[Example 2]

<Production of Catalyst 2>

Ammonium molybdate, ammonium paratungstate, ammonium metavanadate, and antimony acetate were weighed in such a manner that the composition with respect to $Mo_{12}$ became $V_{3.0}W_{1.2}Cu_{1.5}Sb_{0.50}$, and the materials were mixed in an aqueous solvent of 5.2 times the mass of the ammonium molybdate heated to 95°C. Then, copper sulfate was added to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). To the preliminarily calcined powder (E), 5 mass% of crystalline cellulose and 5 mass% of glass fibers having an average fiber length of 150 μm were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for formation, and the centrifugal acceleration was 26.0 G. Next, main calcination was performed under the conditions of 390°C and four hours to obtain a spherical catalyst 2 of the present invention.

[Example 3]

<Production of Catalyst 3>

Ammonium molybdate, ammonium paratungstate, ammonium metavanadate, metallic copper powder, and antimony acetate were weighed in such a manner that the composition with respect to $Mo_{12}$ became $V_{3.0}W_{1.2}Cu_{1.2}Sb_{0.25}$, and the materials were mixed in an aqueous solvent of 5.2 times the mass of the ammonium molybdate heated to 95°C. Then, copper sulfate was added to obtain a prepared liquid (A). The metallic copper powder used was copper (powder) manufactured by KANTO CHEMICAL CO., INC. (standard: Cica grade 1, particle size: 75 μm to 150 μm), and the amount used was an amount resulting in a molar ratio of Cu of 0.25 with respect to $Mo_{12}$. Copper sulfate was used for the amount corresponding to remaining 0.95. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). To the preliminarily calcined powder (E), 5 mass% of crystalline cellulose and 5 mass% of glass fibers having an average fiber length of 150 μm were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for formation, and the centrifugal acceleration was 26.0 G. Next, main calcination was performed under the conditions of 390°C and four hours to obtain a spherical catalyst 3 of the present invention.

[Example 4]

<Production of Catalyst 4>

Ammonium molybdate, ammonium paratungstate, ammonium metavanadate, and antimony acetate were weighed in such a manner that the composition with respect to $Mo_{12}$ became $V_{3.0}W_{1.2}Cu_{1.2}Sb_{0.50}$, and the materials were mixed in an aqueous solvent of 5.2 times the mass of the ammonium molybdate heated to 95°C. Then, copper sulfate was added to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). To the preliminarily calcined powder (E), 5 mass% of crystalline cellulose and 5 mass% of glass fibers having an average fiber length of 150 μm were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for formation, and the centrifugal acceleration was 26.0 G. Next, main calcination was performed under the conditions of 390°C and four hours to obtain a spherical catalyst 4 of the present invention.

[Comparative Example 1]

<Production of Catalyst 5>

Ammonium molybdate, ammonium paratungstate, ammonium metavanadate, and metallic copper powder were weighed in such a manner that the composition with respect to $Mo_{12}$ became $V_{3.0}W_{1.2}Cu_{1.2}$, and the materials were mixed in an aqueous solvent of 5.2 times the mass of the ammonium molybdate heated to 95°C. Then, copper sulfate was added to obtain a prepared liquid (A). The metallic copper powder used was copper (powder) manufactured by KANTO CHEMICAL CO., INC. (standard: Cica grade 1, particle size: 75 $\mu$m to 150 $\mu$m), and the amount used was an amount resulting in a molar ratio of Cu of 0.50 with respect to Mo12. Copper sulfate was used for the amount corresponding to remaining 0.70. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). To the preliminarily calcined powder (E), 5 mass% of crystalline cellulose and 5 mass% of glass fibers having an average fiber length of 150 $\mu$m were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for formation, and the centrifugal acceleration was 26.0 G. Next, main calcination was performed under the conditions of 390°C and four hours to obtain a spherical catalyst 5 of the present invention.

[Comparative Example 2]

<Production of Catalyst 6>

Ammonium molybdate, ammonium paratungstate, ammonium metavanadate, and metallic molybdenum powder were weighed in such a manner that the composition with respect to $Mo_{12}$ became $V_{3.0}W_{1.2}Cu_{1.2}$, and the materials were mixed in an aqueous solvent of 5.2 times the mass of the ammonium molybdate heated to 95°C. Then, copper sulfate was added to obtain a prepared liquid (A). The metallic molybdenum powder used was molybdenum manufactured by SIGMA-ALDRICH (powder, 1-5 $\mu$m, ≥99.9% trace metals basis), and the amount used was in such a manner that the amount corresponding to a molar ratio of 0.50 with respect to Mo12 was the amount of metallic molybdenum and that the amount corresponding to remaining 11.5 was the amount of ammonium molybdate. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). To the preliminarily calcined powder (E), 5 mass% of crystalline cellulose and 5 mass% of glass fibers having an average fiber length of 150 $\mu$m were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for formation, and the centrifugal acceleration was 26.0 G. Next, main calcination was performed under the conditions of 390°C and four hours to obtain a spherical catalyst 6 of the present invention.

[Comparative Example 3]

<Production of Catalyst 7>

Ammonium molybdate, ammonium paratungstate, ammonium metavanadate, and antimony acetate were weighed in such a manner that the composition with respect to $Mo_{12}$ became $V_{3.0}W_{1.2}Cu_{1.0}Sb_{1.0}$, and the materials were mixed in an aqueous solvent of 5.2 times the mass of the ammonium molybdate heated to 95°C. Then, copper sulfate was added to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). To the preliminarily calcined powder (E), 5 mass% of crystalline cellulose and 5 mass% of glass fibers having an average fiber length of 150 μm were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for formation, and the centrifugal acceleration was 26.0 G. Next, main calcination was performed under the conditions of 390°C and four hours to obtain a spherical catalyst 7 of the present invention.

Oxidation reaction was carried out using the obtained catalysts 1 to 7. A reaction tube having an inner diameter of 28.4 mm was filled with 67.6 ml of the catalyst, and a gas having the following composition in which oxygen and nitrogen were added to a gas obtained by catalytic gas-phase oxidation of propylene using a molybdenum-bismuth-based catalyst was introduced. A reaction was performed at SV (space velocity; flow rate of raw material gas per unit time/apparent volume of packed catalyst) of 1020/hr and reaction bath temperature of 260°C.

| | |
|---|---|
| Acrolein | 5.9 vol% |
| Unreacted propylene + other organic compound | 1.7 vol% |
| Oxygen | 4.7 vol% |
| Steam | 17.2 vol% |
| Nitrogen-containing inert gas | 70.5 vol% |

The reaction results obtained by the oxidation reaction are shown in Table 1, and the widths at half height of the peaks in the X-ray diffraction patterns are shown in Table 2 and Table 3. Table 3 shows reference data.

[Table 1]

| Catalyst Name | Raw Material Conversion Rate (%) | Selectivity (%) | Yield (%) |
|---|---|---|---|
| Catalyst 1 | 98.9 | 97.6 | 96.6 |
| Catalyst 2 | 98.6 | 97.8 | 96.4 |
| Catalyst 3 | 96.1 | 97.8 | 94.0 |
| Catalyst 4 | 95.4 | 96.9 | 92.4 |
| Catalyst 5 | 78.9 | 98.3 | 77.6 |
| Catalyst 6 | 87.4 | 97.9 | 85.6 |
| Catalyst 7 | 95.2 | 98.2 | 93.5 |

[Table 2]

| Catalyst Name | Width at Half Height of 22.2° $\pm$ 0.3° (°) |
|---|---|
| Catalyst 1 | 1.02 |
| Catalyst 2 | 1.00 |
| Catalyst 3 | 1.32 |
| Catalyst 4 | 1.48 |
| Catalyst 5 | 0.63 |
| Catalyst 6 | 0.64 |
| Catalyst 7 | 1.62 |

[Table 3]

| Catalyst Name | Width at Half Height of 8.3° $\pm$ 0.5° (°) | Width at Half Height of 26.7° $\pm$ 0.3° (°) | Width at Half Height of 45.4° $\pm$ 0.3° (°) |
|---|---|---|---|
| Catalyst 1 | 2.32 | 6.00 | 0.97 |
| Catalyst 2 | 2.52 | 6.18 | 0.94 |
| Catalyst 3 | 2.38 | 5.42 | 1.02 |

(continued)

| Catalyst Name | Width at Half Height of 8.3° ± 0.5° (°) | Width at Half Height of 26.7° ± 0.3° (°) | Width at Half Height of 45.4° ± 0.3° (°) |
|---|---|---|---|
| Catalyst 4 | 2.83 | 5.02 | 0.98 |
| Catalyst 5 | 2.61 | 7.41 | 0.63 |
| Catalyst 6 | 2.45 | 7.24 | 0.66 |
| Catalyst 7 | 2.35 | 4.65 | 1.04 |

From the results in Table 1 and Table 2, it was found that the catalysts of the present invention had a high raw material conversion rate, namely, a high catalytic activity.

[Reference Example 3]

The catalyst described in Example 1 of PTL 1 was prepared according to the description of the Example of PTL 1. That is, ammonium tungstate, ammonium metavanadate, ammonium molybdate, antimony trioxide powder, and copper sulfate were used as raw materials to obtain a catalyst having a catalytically active component having a composition $V_3W_{1.2}Cu_{1.2}Sb_{0.5}$ with respect to $Mo_{12}$. When the width at half height of the obtained catalyst was measured by the method described in the present invention, the width at half height at 22.2° ± 0.3° in the X-ray diffraction pattern was 0.52. Oxidation reaction was carried out by the method described in the present invention, and the results were a reaction temperature of 260°C, a raw material conversion rate of 19.6%, selectivity of 96.3%, and a yield of 18.9%.

The present application is based on Japanese Patent Application No. 2023-150163 filed on September 15, 2023, contents of which are incorporated herein by reference.

INDUSTRIAL APPLICABILITY

According to the present invention, the catalytic activity can be maintained high when an unsaturated carboxylic acid is produced by subjecting an unsaturated aldehyde as a raw material to a catalytic gas-phase oxidation reaction. Therefore, thermal deterioration of the catalyst for producing acrylic acid can be prevented, and the plant can be stably operated for a long period of time, which is very useful.

**Claims**

1.   A catalyst for producing an unsaturated carboxylic acid, wherein a catalytically active component has a composition represented by the following formula (1), and a width at half height of a peak at 22.2° ± 0.3° in an X-ray diffraction pattern obtained using CuKα ray as an X-ray source is 0.65° or more and 1.60° or less,

$$(Mo)_{12}(V)_a(W)_b(Cu)_c(Sb)_d(X)_e(Y)_f(Z)_g(O)_h \qquad (1)$$

(in the formula (1), Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of niobium, cerium, tin, chromium, manganese, iron, cobalt, samarium, germanium, titanium, and arsenic, a, b, c, d, e, f, g, and h represent atomic ratios of the respective elements, $0 < a \le 10.0$, $\le b \le 10.0$, $0 < c \le 6.0$, $\le d \le 10.0$, $\le e \le 0.50$, $\le f \le 1.0$, and $0 \le g < 6.0$ are satisfied with respect to molybdenum atom being 12, and h is the number of oxygen atoms necessary for satisfying the valence of each component.)

2.   The catalyst for producing an unsaturated carboxylic acid according to claim 1, wherein in the formula (1), $1.0 \le a \le 5.0$, $0.50 \le b \le 3.0$, $0.50 \le c \le 3.0$, and $< d \le 2.0$ are satisfied.

3.   The catalyst for producing an unsaturated carboxylic acid according to claim 1 or 2, wherein the catalytically active component is carried on an inert carrier.

4.   The catalyst for producing an unsaturated carboxylic acid according to claim 3, wherein the inert carrier is silica, alumina, or a combination thereof.

5. A method for producing an unsaturated carboxylic acid using the catalyst for producing an unsaturated carboxylic acid according to claim 1 or 2.

6. A method for producing an unsaturated carboxylic acid using a reaction tube filled with two or more types of the catalyst for producing an unsaturated carboxylic acid according to claim 1 or 2 in multiple layers.

# FIG. 1

## INTERNATIONAL SEARCH REPORT

| International application No. |
|---|
| **PCT/JP2024/029661** |

**A. CLASSIFICATION OF SUBJECT MATTER**

*B01J 23/888*(2006.01)i; *C07B 61/00*(2006.01)i; *C07C 51/235*(2006.01)i; *C07C 57/055*(2006.01)i
FI: B01J23/888 Z; C07C57/055 A; C07C51/235; C07B61/00 300

According to International Patent Classification (IPC) or to both national classification and IPC

**B. FIELDS SEARCHED**

Minimum documentation searched (classification system followed by classification symbols)

B01J21/00-38/74; C07B61/00; C07C51/235; C07C57/04; C07C57/055

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JST7580/JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2018-43197 A (NIPPON KAYAKU KABUSHIKI KAISHA) 22 March 2018 (2018-03-22) paragraphs [0001], [0033]-[0035], fig. 1 | 1-5 |
| Y | | 6 |
| Y | JP 2023-80395 A (NIPPON KAYAKU KABUSHIKI KAISHA) 09 June 2023 (2023-06-09) paragraph [0034] | 6 |
| A | JP 8-299797 A (NIPPON KAYAKU KABUSHIKI KAISHA) 19 November 1996 (1996-11-19) entire text, all drawings | 1-6 |
| A | WO 2022/050110 A1 (NIPPON SHOKUBAI CO., LTD.) 10 March 2022 (2022-03-10) entire text | 1-6 |
| A | WO 2010/038676 A1 (NIPPON SHOKUBAI CO., LTD.) 08 April 2010 (2010-04-08) entire text | 1-6 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **17 September 2024** | **01 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
**Information on patent family members**

International application No.

**PCT/JP2024/029661**

| Patent document cited in search report | | | Publication date (day/month/year) | Patent family member(s) | | | Publication date (day/month/year) |
|---|---|---|---|---|---|---|---|
| JP | 2018-43197 | A | 22 March 2018 | (Family: none) | | | |
| JP | 2023-80395 | A | 09 June 2023 | (Family: none) | | | |
| JP | 8-299797 | A | 19 November 1996 | US | 5959143 | A | |
| | | | | entire text, all drawings | | | |
| | | | | EP | 1375466 | A1 | |
| | | | | WO | 1996/027437 | A1 | |
| | | | | CN | 1148352 | A | |
| WO | 2022/050110 | A1 | 10 March 2022 | EP | 4209268 | A1 | |
| | | | | entire text | | | |
| | | | | KR | 10-2023-0036136 | A | |
| | | | | CN | 116033968 | A | |
| WO | 2010/038676 | A1 | 08 April 2010 | US | 2011/0166384 | A1 | |
| | | | | entire text | | | |
| | | | | EP | 2347823 | A1 | |
| | | | | CN | 102066000 | A | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H08299797 A
- JP 2003251184 A
- JP 2015120133 A
- JP 2018043197 A
- JP 2001079408 A
- WO 2012073584 A
- JP 2009214105 A
- JP 2015096497 A
- WO 2020196150 A
- JP 2011516378 A
- JP 2023150163 A